# EUROPEAN PATENT SPECIFICATION

(11) **EP 0 820 589 B1**
(45) Date of publication and mention of the grant of the patent: **04.06.2003**
(21) Application number: 96910258.1
(22) Date of filing: 26.03.1996
(51) Int. Cl.: G01N 33/49

(54) **METHOD AND ARRANGEMENT FOR TESTING ANTI-COAGULATED FULL BLOOD**
VERFAHREN UND VORRICHTUNG ZUR UNTERSUCHUNG VON ANTI-KOAGULIERTEM BLUT
METHODE ET DISPOSITIF DU SANG ANTI-COAGULE

(30) Priority: 10.04.1995 SE 9501320
(43) Date of publication of application: 28.01.1998
(73) Proprietor: JÄREMO, Petter, 603 56 Norrköping (SE)
(72) Inventor: JÄREMO, Petter, 603 56 Norrköping (SE)
(74) Representative: Lautmann, Kurt O.
(86) International application number: SE9600379
(87) International publication number: WO96032639

(56) References cited:
- JOURNAL OF LABORATORY AND CLINICAL MEDICINE, Volume 103, 1984, STEIN HOLME et al., "Studies of the Platelet Density Abnormality in Myeloproliferative Disease".
- BRITISH JOURNAL OF HAEMATOLOGY, Volume 55, 1983, B. BONEU et al., "Platelet Density Analysis: a Tool for the Detection of Acquired Storage Pool Disease in Man", pages 523-532.
- MEDICAL & BIOLOGICAL ENGINEERING & COMPUTING, Volume 26, March 1988, P. JAEREMO, "Computerised Method for Monitoring Stored Platelet Packs", pages 193-198.

## Description

During medical treatment blood is drawn from a patient for testing in various apparatuses in order to diagnose certain states in the patient. The blood contains red corpuscles, white corpuscles, thrombocyte cells and blood plasma. A blood test reveals the sedimentation rate, the value of which provides certain information about a patient. It would be desirable if a method and arrangement were available at every hospital or medical station, enabling rapid diagnosis of whether a patient has a myeloproliferative disease or is in the risk zone for cardiac infarction, pulmonary embolism and/or venous thrombosis.

It should be explained that thrombocytes (platelets) constitute the blood cells that initiate coagulation. The thrombocytes can be divided into different populations and dense thrombocytes are considered to be more reactive than those that are less dense. An increased thrombocyte density, in comparison with a normally healthy person, indicates an increased risk of suffering cardiac infarction, pulmonary embolism or venous thrombosis. If an increased quantity of less dense thrombocytes occur in the circulation in comparison with a normally healthy person, it indicates that the bone marrow is unable to form thrombocytes of normal quality. This is the case with various myeloproliferative diseases such as essential thrombocythemia. Since these diseases have serious complications, such as haemorrhaging, embolism and progress to acute leukaemia, they should be treated with cytotoxins. It is important that the diagnosis is adequate in view of the side effects caused by cytotoxins.

A method for determining platelet density distributions is described by Bonen et al. (British Journal of Haematology, 1983, 55, 523-532), Plasma samples are centrifuged on a discontinuous density gradient, and the fractions areCollected separately.

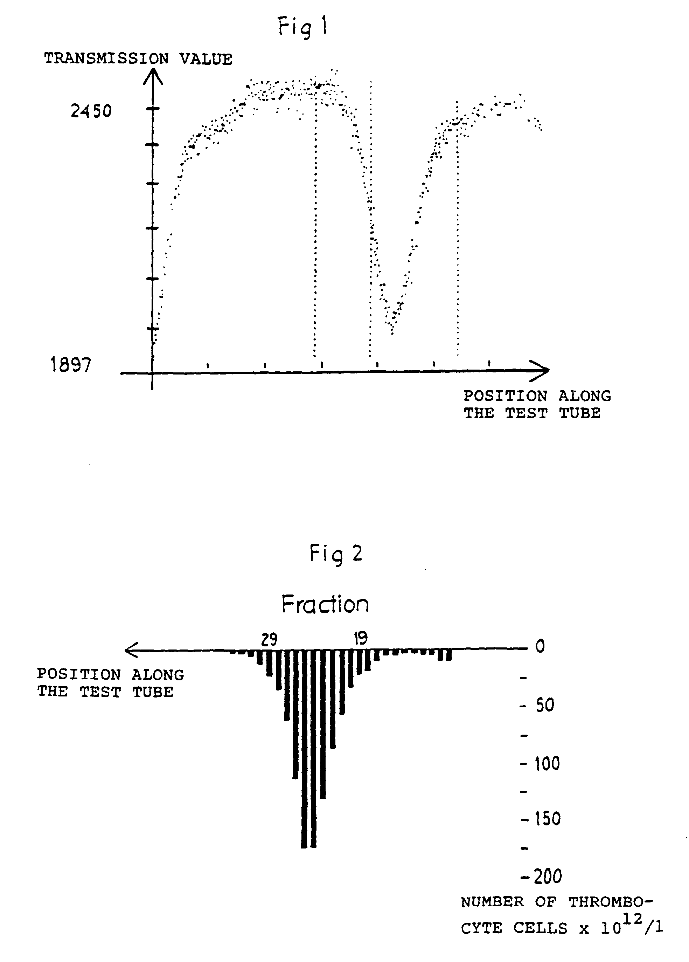

The present invention has managed to provide a method and an arrangement by means of which a curve is obtained, as shown in Figure 1. In this Figure the x-axis indicates the density, and thus indirectly the thrombocyte distribution in the gradient, and the γ-axis the value of the light intensity. Characteristic of said curve is that it consists of one or more consecutive, upwardly directed curve arcs between which downwardly directed points or peaks are formed. It has now been discovered that a peak located to the left along the x-axis is an indication of low density in the thrombocytes or platelets. This is thus a strong indication that the patient has a myeloproliferative disease, e.g. essential thrombocythemia. This disease may then develop into acute leukaemia and the condition also entails complications such as thrombocyte formation and bleeding. The disease thus often requires treatment with cytotoxic drugs. Since cytotoxic treatment entails complications it is desirable for the state of "essential thrombocythemia" to be diagnosed as adequately as possible. A peak located more to the right indicates that the patient has more reactive thrombocytes and thus runs a greater risk of cardiac infarction, pulmonary embolism and/or venous thrombosis. If the patient has already suffered a cardiac infarction, a peak located more to the right indicates more reactive thrombocytes and thus an increased risk of having another cardiac infarction. In the case of myeloproliferative disease e.g. essential thrombocythemia or polycythemia vera, and thus increased risk of progression to malign leukaemia, said peaks are located more to the left. The thrombocyte ratio in the blood of a patient is utilized in order to obtain said curve. It is a fact that a patient has a number of thrombocytes that do not have the same density. The total thrombocyte population thus consists of a mixture of thrombocytes with different density. The object of the invention is to sort said thrombocytes so that thrombocytes of the same density are collected. This is achieved by mixing two solutions of Percoll ® having different densities in a test tube. A gradient is obtained in the test tube, without centrifuging, which has a density range of 1.04 kg/l to 1.09 kg/l. A quantity of full blood drawn from the patient is added to the test tube as a layer on top of the gradient. Full blood means that all the constituents are present and that it has been mixed with a constituent which inactivates the thrombocytes in the blood. The Percoll and blood are centrifuged and the various thrombocyte cells and mono-nuclear white cells, e.g. monocytes and lymphocytes, will be located in the relevant gradient layer in the test tube, while the remaining white and the red corpuscles will collect at the bottom of the test tube. A light beam is passed successively linearly from one end to the other end of the test tube. The light beam through the test tube has constant intensity in the form of white halogen light produced by a halogen lamp, passes through the test tube and is received by a light sensor. The values emitted by the light sensor pass to a computer which utilizes the values obtained to produce the curve shown in Figure 1. When the test has been completed "Density marker Beads" made by Pharmacia are added to the test tube. This is then centrifuged and three density bars are obtained, a green, a red and a blue one, located where the gradient density is 1.054, 1.062 and 1.078 kg/l. The bar transitions are marked and the test tube then emptied. The test tube with said markings is then subjected to the same illumination as the test with the thrombocytes so that a test curve is obtained. The values obtained are utilized by the computer unit so that the curve shown in Figure 1 is compared with the calibration curve. This is performed in a computer which calculates the correct peak values.

Density marker beads consist of dextrane molecules. Density marker beads are particles (exactly like thrombocytes) and since they are much larger than thrombocytes a much shorter centrifuge time is required before they reach equilibrium. Green macromolecules (beads) have a density of 1.051, red macromolecules (beads) have a density of 1.062 and blue macromolecules (beads) have a density of 1.077 (all kg/ l).

It has thus been discovered that a curve according to Figure 1 can be quickly obtained which enables a medical reception to quickly determine whether a patient has a myeloproliferative disease (e.g. essential thrombocythemia) which can develop into acute leukaemia and which must therefore be treated with cytotoxins or is in the risk zone of developing cardiac infarction, pulmonary embolism and/or deep venous thrombosis. Additional characteristics of the present invention are revealed in the appended claims.

The present invention will be discussed in more detail with reference to the accompanying drawings in which,
- Figure 1: shows an information curve of the light transmission through a test tube containing density-separated thrombocytes,
- Figure 2: shows the thrombocyte distribution in the gradient, the light transmission pattern of which is shown in Figure 1
- Figure 3: shows an apparatus for measuring the thrombocyte distribution,
- Figure 4: shows a test tube with markings marking the positions of Density marker beads, and
- Figure 5: shows a test curve for test liquid and also a part of the curve according to Figure 1.

According to the present method, two Percoll® solutions are produced having densities of 1.09 kg/l and 1.04 kg/l. These two solutions are mixed with ion-free water and both are mixed with a solution consisting of
I. 0.13 mol/l Na₂EDTA and 0.15 mol/l Na₃ citrate (pH 7.4 at 25°C)
II. 1 mg/l prostaglandin E₁ and 1 ml 95% (w/v) ethanol in H₂O
III. 2.7 mmol/l theophylline dissolved in 0.15 mol TRIS chloride buffer (pH 7.4 at 25°C)

Sodium chloride is then added to both the solutions in said mixture so that they both become iso-osmolar. The substances I, II and III are designed to passivate the thrombocyte cells, thereby preventing artefacts in vitro (e.g. activation and thus granule liberation which causes density reduction), i.e. in the test tube. The two Percoll® solutions are mixed with the aid of an apparatus (gradient mixer) called model 385, Bio-Rad, producing a density gradient between 1.09 and 1.04. The mixture is supplied to a test tube which is placed in a centrifuge of type Hettich Rotanta/TR which gives rise to centrifuging with 3400 g. Centrifuging is performed so that at the start of a rotation about a centre, the test tube being distanced from the centre, this is raised to a horizontal position with the bottom of the test tube facing out, whereupon the test tube is subjected to said radial force upon rotation. This is effected with a swing out rotor. Prior to being centrifuged 15 ml full blood from a patient is added to the test tube. Said 15 ml blood is mixed with 5 ml of liquid I, II and III, so that the thrombocytes in the blood become inactive. Said blood is now added to the test tube and forms a layer above the liquid already there. The whole is centrifuged for 1.5 hour with a radial force of 3400 g. The centrifuging causes the thrombocytes to move to the positions in the gradient corresponding to their density. The heavier white and red blood corpuscles arrive at the bottom of the test tube. Density marker beads are added and the test tube centrifuged for five minutes, after which the bar transitions are marked on the outside of the test tube.

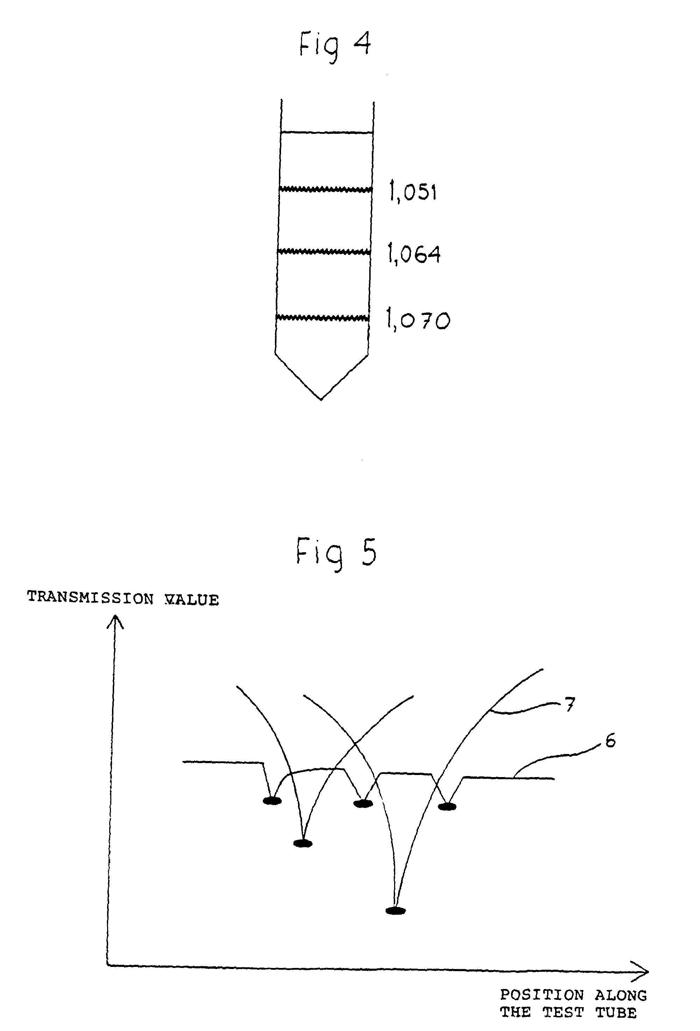

The various thrombocytes have by now placed themselves in the layers where the liquid has the same density. The test tube is secured in some way to the plate of a stepping motor. The plate is so designed that, controlled by computers, it is moved up and down by the stepping motor and the movement of the stepping motor, i.e. position, is transmitted to a computer 1 where the computer clearly indicates the exact level of the test tube. The platform is adjusted by the stepping motor 2 so that the upper end of the test tube is located at the same level as a light source 4 emitting halogen light. A light-sensitive body is located on the other side of the test tube to receive the light passing through the test tube. When a measuring process is commenced the test tube is so placed in relation to the light source that the light source is at the upper end of the test tube. The test tube is then gradually lifted by the stepping motor and during this movement the light passing through the test tube is registered and reproduced in the curve in Figure 1. Of course, the relative movement between light source and test tube may also be achieved by the test tube remaining stationary while the light source is moved. In Figure 3, 4 denotes the light source and 5 the light-sensitive part. 1 is the computer unit with display screen, and 3 is the unit controlling the stepping motor 2. Figure 3 deserves a somewhat more detailed description. In the figure, 1 is a computer with a screen. The computer cooperates with a holder 3 for suitable printed circuit boards. The stepping motor 2 receives its impulses via the printed circuit boards. The drive shaft of the stepping motor consists of a screw cooperating with a tube having internal threading, said tube being controlled so that it can only be moved up or down but cannot be turned. The tube has a platform on which a test tube containing a gradient solution is secured in some known manner, i.e. the gradient solution has contributed to thrombocytes being arranged along the test tube according to their densities, so that after centrifuging the thrombocyte density varies linearly from one end of the liquid to the other end. The other ingredients of the blood are collected at the bottom of the test tube. White light from a halogen lamp 4 is passed through the test tube. The white light is allowed to pass through the liquid in the test tube and a light sensor 5 is arranged diametrically opposite, on the other side of the test tube, to receive light which has passed through the test tube and transmit it to the computer. Density marker beads may also have been added to the liquid in the test tube, together with the full blood, so that the liquid in the test tubes acquires three clear colour transitions. The colours are red, blue and green. These colour transitions are suitably marked with two peripheral lines on the outside of the test tube. The platform for the test tube is adjusted by the stepping motor 2 so that the light from the lamp 4 shines through the uppermost part of the liquid. The platform with the test tube is then moved stepwise up, each step or position being marked by the computer, as well as the light that passes through the test tube at the position. When the test tube has been moved up so far that its bottom has been illuminated, the curve according to Figure 1 with its downward peaks is obtained. To obtain correct values on said curve, the test tube is moved from the position where illumination of the liquid content in the upper liquid level of the test tube occurs, to the bottom of the test tube in such a manner that said peripheral lines are marked, i.e. a test curve is obtained of the type shown in Figure 5 which has been designated 6. Figure 5 also shows the peak part of the curve in Figure 1, designated 7. Said peripheral lines are considered as correct test levels. When these values are supplied to the computer, the latter will calculate the correct value of a peak. One or more of the downwardly directed points or peaks are then utilized to diagnose a pathological state tending towards either cancer or infarction.

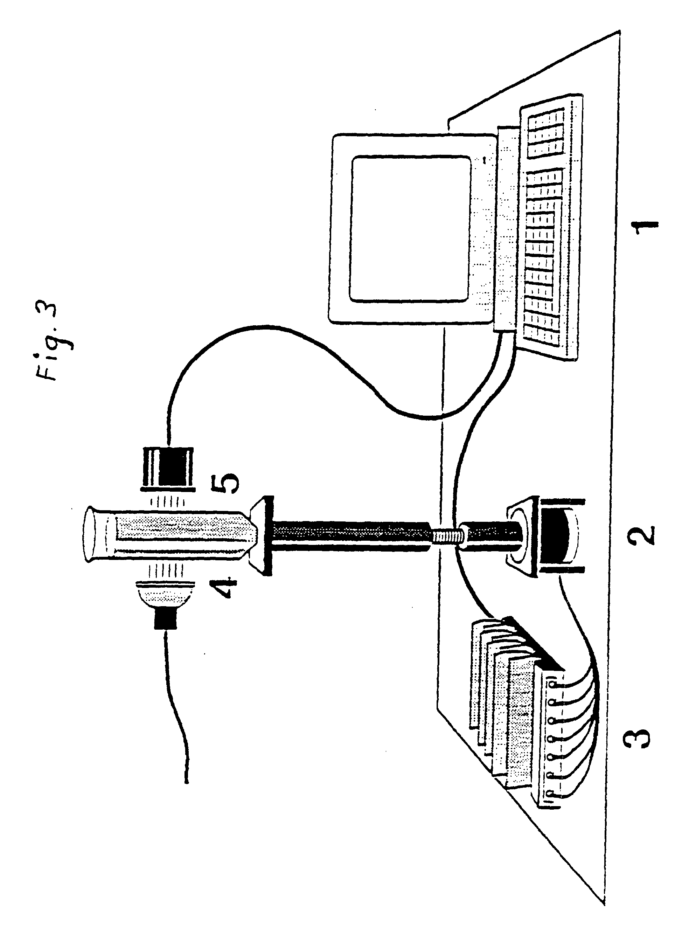

The stacks shown in Figure 2 indicate the number of thrombocyte cells of different density.

This method of determining whether a patient has cancer or is in the risk zone for cardiac infarction, pulmonary embolism and/or venous thrombosis is simple and quick to perform and the apparatus and arrangement is not so expensive. Every hospital should therefore be able to have such an arrangement. The arrangement comprises a mixer for two Percoll solutions, model 385, Bio-Rad, for instance. A centrifuge, constituting standard equipment is also required and may be a Hettich Rotanta/TR with swing out rotor, for instance. These two units are combined with the equipment shown in Figure 3. The three units can then be combined in any suitable manner to provide a convenient apparatus for use in hospitals.

It should be evident that the procedure can be somewhat simplified by producing the two Percoll solutions, inhibitor solution, water and NaCI as a commercial product. The arrangement may also be correctly calibrated, thus dispensing with the need for "Density marker Beads". In this case a doctor need only carry out centrifuging and thereafter perform illumination of the test tube along its length. Centrifuging can be limited to only once if Density marker Beads are added to the full blood.

The curve described above may also be given such a character that the peaks point upwards. Irrespective of the nature of the curve it is always the same density situation that is reproduced.

## Claims

1. A method of testing anti-coagulated full blood, for the purpose of ascertaining a state such a myeloproliferative disease (e.g. essential thrombocythemia), the risk zone for cardiac infarction, pulmonary embolism or venous thrombosis, using a gradient solution and centrifugation,
**characterized in that** a quantity of full blood is supplied to a container such as a test tube containing a gradient solution, preferably as a layer on top of the gradient solution, that the container is subjected to centrifugation so that the thrombocytes in the blood acquire positions in the liquid corresponding to the density of the thrombocytes, that light, preferably white light, is caused to pass through the container, that the light which has passed through is caught by a light-sensitive unit, that a relative movement is effected between the light source cooperating with the light-sensitive unit and the container, from the surface of the liquid in the container to the bottom of the container, and that data concerning the received light intensity of said light-sensitive unit and position data for the light intensity are supplied to a computer which utilizes said data to plot a curve with one or more downwardly directed points, where the position of said points along a position axis is indicative of the prevailing state of health.

2. A method as claimed in claim 1,
**characterized in that** an inhibiting solution is added to the gradient solution in order to inactivate the thrombocytes.

3. A method as claimed in claims 1 or 2,
**characterized in that** sodium chloride is added to the gradient solution so that it becomes iso-osmolar.

4. A method as claimed in claim 1,
**characterized in that** density marker beads are supplied to the solution, either directly or mixed with the full blood, this addition forming three colour bars coloured green, blue and red, said bars indicating the correct density values.

5. A method as claimed in any of the preceding claims,
**characterized in that** the gradient solution is formed of a mixture of two different Percoll® solutions having different density, such as 1.09 kg/l and 1.04 kg/l.

6. A method as claimed in any of the preceding claims,
**characterized in that** the thrombocytes are passivated by a solution containing:
I 0.13 mol/l Na₂EDTA and 0.15 mol/l Na₃ citrate (pH 7.4 at 25° C)
ll 1 mg/l prostaglandin E₁ and i ml95% (w/v) ethanol in H₂O
lll 2.7 mmol/l theophylline dissolved in 0.15 mol TRIS chloride buffer (pH 7.4 at 25° C)

7. An arrangement for performing a method according to one or more of the preceding claims, containing a centrifugal unit and means for supplying a gradient solution,
**characterized in that** it comprises a unit for obtaining light-intensity data and position data for the various density data, said unit consisting of a holder for said container, which holder is movable in vertical direction by means of a stepping motor indicating position data, a light source emitting white light such as halogen light through said container, which light source cooperates with a light-sensitive unit located on the other side of the container and indicating light-intensity data, and a computer receiving said two types of data, evaluating said data and providing information concerning state of health via a display screen, for instance, which is able to reproduce a curve having one or more downwardly directed points, the position(s) of the point(s) providing the desired information.

8. An arrangement as claimed in claim 7,
**characterized in that** it includes a centrifuge in order to achive gradient separation in the container when it is filled with liquid and then between the surface of the liquid and the bottom.

9. An arrangement as claimed in claim 7,
**characterized in that** it includes apparatus for producing a liquid with gradient properties.

10. An arrangement as claimed in claim 7,
**characterized in that** the stepping motor has a vertical shaft extending upwards, the shaft extension being provided with a thread cooperating with an internal thread in a hole in the unit acting as holder for liquid container.

## Patentansprüche

1. Verfahren zum Testen von antikoaguliertem Vollblut zum Zwecke des Ermittelns eines Zustandes wie einer myeloproliferativen Erkrankung (beispielsweise essentieller Thrombozytämie), den Risikobereich für Herzinfarkt, Lungenembolie oder venöse Thrombose, unter Verwendung einer Gradientenlösung und Zentrifugation,
**dadurch gekennzeichnet, dass** eine Menge an Vollblut in einen Behälter, wie z.B. ein Teströhrchen, mit einer Gradientenlösung gegeben wird, vorzugsweise als Schicht oben auf die Gradientenlösung, dass der Behälter einer Zentrifugation unterworfen wird, so dass die Thrombozyten des Blutes in der Flüssigkeit Positionen einnehmen, die der Dichte der Thrombozyten entsprechen, dass Licht, vorzugsweise weißes Licht, durch den Behälter geführt wird, dass das dabei durchgefallene Licht von einer lichtempfindlichen Einheit empfangen wird, dass eine relative Bewegung zwischen der mit der lichtempfindlichen Einheit zusammenwirkenden Lichtquelle und dem Behälter bewirkt wird, von der Oberfläche der Flüssigkeit im Behälter bis zum Boden des Behälters, und dass Daten, die die durch die genannte lichtempfindliche Einheit aufgefangene Lichtintensität betreffen, und Positionsdaten für die Lichtintensität an einen Computer weitergegeben werden, der diese Daten einsetzt, um eine Kurve mit einem oder mehreren nach unten gerichteten Punkten zu plotten, wobei die Position dieser Punkte entlang einer Positionsachse den vorherrschenden Gesundheitszustand anzeigt.

2. Verfahren nach Anspruch 1, **dadurch gekennzeichnet, dass** eine inhibierende Lösung zu der Gradientenlösung zugegeben wird, um die Thrombozyten zu inaktivieren.

3. Verfahren nach einem der Ansprüche 1 oder 2, **dadurch gekennzeichnet, dass** Natriumchlorid zu der Gradientenlösung zugegeben wird, so dass sie iso-osmolar wird.

4. Verfahren nach Anspruch 1, **dadurch gekennzeichnet, dass** der Lösung Dichtemarkierungs-Perlen oder -Kügelchen zugegeben werden, und zwar entweder direkt oder in Mischung mit Vollblut, derart, dass durch diesen Zusatz drei Farbstreifen oder -zonen mit den Farben grün, blau und rot gebildet werden, wobei die Streifen oder Zonen die korrekten Dichtewerte anzeigen.

5. Verfahren nach einem der voranstehenden Ansprüche, **dadurch gekennzeichnet, dass** die Gradientenlösung aus einer Mischung zweier unterschiedlicher Percoll®-Lösungen mit unterschiedlicher Dichte hergestellt wird, beispielsweise mit 1,09 kg/l und 1,04 kg/l.

6. Verfahren nach einem der voranstehenden Ansprüche, **dadurch gekennzeichnet, dass** die Thrombozyten durch eine Lösung passiviert werden, die enthält:
l 0,13 mol/l Na₂EDTA und 0,15 mol/l Na₃Citrat (pH 7,4 bei 25 °C)
II 1 mg/l Prostaglandin E₁ und i ml95% (w/v) Ethanol in H₂O
lll 2,7 mmol Theophyllin, gelöst in 0,15 mol TRIS-chlorid-Puffer (pH 7,4 bei 25 °C).

7. Anordnung zum Durchführen eines Verfahrens nach einem oder mehreren der voranstehenden Ansprüche, enthaltend eine Zentrifugeneinheit und Mittel zum Bereitstellen einer Gradientenlösung, **dadurch gekennzeichnet, dass** sie eine Einheit zum Aufnehmen von Lichtintensitäts-Daten und Positionsdaten für die verschiedenen Dichtedaten umfasst, wobei diese Einheit aus einem Behälter-Halter, der mit Hilfe eines Positionsdaten anzeigenden Schrittmotors in vertikaler Richtung bewegbar ist, eine weißes Licht wie z. B. Halogenlicht durch den genannten Behälter emittierende Lichtquelle, die mit einer lichtempfindlichen, auf der anderen Seite des Behälters befindlichen und Lichtintensitäts-Daten anzeigenden Einheit zusammenwirkt, und einem Computer besteht, der die beiden Arten von Daten aufnimmt, diese Daten auswertet und Informationen bezüglich des Gesundheitszustandes über einen Anzeigeschirm ausgibt, der beispielsweise in der Lage ist, eine Kurve mit einer oder mehreren nach unten gerichteten Punkten zu reproduzieren, wobei die Position(en) des Punktes oder der Punkte die gewünschte Information liefem.

8. Anordnung nach Anspruch 7, **dadurch gekennzeichnet, dass** sie eine Zentrifuge umfasst, um eine Gradientenseparation in dem mit Flüssigkeit gefüllten Behälter, und zwar zwischen der Flüssigkeitsoberfläche und dem Boden, zu erzielen.

9. Anordnung nach Anspruch 7, **dadurch gekennzeichnet, dass** sie eine Vorrichtung zum Erzeugen einer Flüssigkeit mit Gradienteneigenschaften umfasst.

10. Anordnung nach Anspruch 7, **dadurch gekennzeichnet, dass** der Schrittmotor einen vertikalen Schaft aufweist, der sich nach oben erstreckt, wobei der Schaft über seine Länge mit einem Gewinde versehen ist, das mit einem Innengewinde in einem Loch in der Einheit zusammenwirkt, die als Halter für den Flüssigkeitsbehälter fungiert.

## Revendications

1. Méthode pour tester le sang total anti-coagulé dans le but d'établir un état comme une maladie myéloproliférative (par ex. la thrombocytopénie essentielle), la zone à risque d'infarctus cardiaque, d'embolie pulmonaire ou de thromboses veineuses, à l'aide d'une solution en gradient de concentration et de centrifugation, **caractérisée en ce que**
une certaine quantité du sang total est mise dans un conteneur du genre tube à essais contenant une solution en gradient, de préférence sous forme d'une couche sur le sommet de cette solution en gradient, le conteneur étant soumis à centrifugation de manière que les thrombocytes dans le sang atteignent des positions dans le liquide qui correspondent à la densité des thrombocytes, que l'on fait passer de la lumière, de préférence une lumière blanche à travers le conteneur, la lumière l'ayant traversée étant saisie par une unité sensible à la lumière, qu'un mouvement relatif est effectué entre la source de lumière coopérant avec l'unité sensible à la lumière et le conteneur, à partir de la surface du liquide dans le conteneur jusqu'au fond du conteneur et **en ce que** les données concernant l'intensité de la lumière reçue de ladite unité sensible à la lumière et les données de position de l'intensité de la lumière sont saisies dans un ordinateur lequel utilise lesdites données pour tracer une courbe avec un ou plusieurs points dirigés vers le bas, où la position desdits points le long d'un axe de positions montre l'état de santé actuel

2. Méthode selon la revendication 1,
**caractérisée en ce qu'**une solution inhibante est ajoutée à la solution en gradient pour inactiver les thrombocytes.

3. Méthode selon la revendication 1 ou 2,
**caractérisée en ce que** du chlorure de sodium est apporté à la solution en gradient de manière qu'elle devienne iso-osmolaire.

4. Méthode selon la revendication 1,
**caractérisée en ce que** des billes marqueurs de densité sont apportées à la solution, soit directement soit par mélange avec le sang total, cet apport formant trois bâtons colorés de couleur verte, bleue et rouge, lesdits bâtons indiquant les valeurs de densité correctes.

5. Méthode selon l'une des revendications susmentionnées,
**caractérisée en ce que** la solution en gradient est composée d'un mélange de deux différentes solutions de Percoll® ayant des densités différentes, notamment 1,09 kg/l et 1,04 kg/l.

6. Méthode selon l'une des revendications susmentionnées,
**caractérisée en ce que** les thrombocytes sont passivés au moyen d'une solution contenant :
I 0,13 mol/l de Na₂EDTA et 0,15 mol/l de citrate de Na₃ (pH 7,4 à 25°C)
II 1 mg/l de prostaglandine E₁ et 1 ml d'éthanole à 95% (v/p) dans du H₂O
lll 2,7 mmol/l de théophylline dissoute dans un tampon de chlorure de tris de 0,15 mol (pH 7,4 à 25°C).

7. Dispositif pour la réalisation d'une méthode selon l'une ou plusieurs des revendications susmentionnées, contenant une unité centrifuge et des moyens pour apporter une solution en gradient,
**caractérisé en ce qu'**il comprend une unité pour l'obtention des données de l'intensité de lumière et des données de position pour les différentes données de densité, ladite unité étant constituée d'un support pour ledit conteneur, ce support étant déplaçable dans le sens vertical au moyen d'un moteur pas à pas indiquant les données de position, une source de lumière émettant une lumière blanche telle que la lumière halogène à travers ledit conteneur, cette source de lumière coopérant avec une unité sensible à la lumière placée de l'autre côté du conteneur et indiquant les données de sensibilité à la lumière et un ordinateur recevant lesdits deux types de données, qui évalue lesdites données et fournit des informations concernant l'état de santé via, par exemple, un écran d'affichage lequel est capable de reproduire une courbe ayant un ou plusieurs points dirigés vers le bas, la (les) position(s) du (des) point(s) fournissant les informations désirées.

8. Dispositif selon la revendication 7,
**caractérisé en ce qu'**il comprend un centrifuge pour assurer une séparation en gradient dans le conteneur lorsqu'il est rempli de liquide et ensuite entre la surface du liquide et le fond.

9. Dispositif selon la revendication 7,
**caractérisé en ce qu'**il comprend un appareil pour produire un liquide avec des propriétés de gradient.

10. Dispositif selon la revendication 7,
**caractérisé en ce que** le moteur pas à pas dispose d'un arbre vertical se prolongeant vers le haut, le prolongement de l'arbre étant pourvu d'un filetage coopérant avec un taraudage interne d'un alésage dans l'unité servant de support pour le conteneur du liquide.
